# EUROPEAN PATENT APPLICATION

(11) **EP 4 835 908 A1**
(43) Date of publication of application: **04.11.2026**
(21) Application number: 26176012.8
(22) Date of filing: 30.04.2026
(51) Int. Cl.: G16H 70/00, G16H 10/20, G16H 10/60, G16H 50/20, G16H 70/20

(54) **MULTI-AGENT HEALTHCARE KNOWLEDGE PLATFORM WITH CO-OCCURRENCE-DRIVEN INTELLIGENCE**

(30) Priority: 30.04.2025 US 202563797637 P
(71) Applicant: Suhardi, Nicolas, New York, NY 10021 (US); Suhardi, Vincentius, New York, NY 10021 (US)
(72) Inventor: Suhardi, Nicolas, New York, NY 10021 (US); Suhardi, Vincentius, New York, NY 10021 (US)
(74) Representative: Berggren Oy

(57) **Abstract**

A healthcare knowledge platform is provided herein. The healthcare knowledge platform includes a user interface configured to receive queries, a multi-agent processing module having a directing agent, a conversational agent, and specialized agents for knowledge retrieval, summarization, and co-occurrence-based recommendations, a knowledge retrieval engine configured to access at least one source selected from large language models, curated healthcare databases, peer-reviewed journals, textbooks, and electronic health record (EHR)-based co-occurrence data, a summarizing engine configured to generate summarized content or perform live meta-analyses of retrieved documents, a dynamically expandable healthcare knowledge graph storing medical concepts, relationships, metadata, and co-occurrence frequencies, and a database for storing user queries, retrieved data, summaries, co-occurrence metrics, and system analytics.

## Description

### STATEMENT REGARDING FEDERALLY SPONSORED RESEARCH OR DEVELOPMENT

Not Applicable.

### INTRODUCTION

Healthcare systems have undergone significant technological advancements in recent years, with the integration of artificial intelligence (AI) and machine learning techniques becoming increasingly prevalent. These technologies have been applied to various aspects of healthcare, including clinical decision support, patient data management, and workflow optimization. However, existing solutions often operate in silos, lacking the comprehensive integration necessary to fully leverage the potential of AI in healthcare.

Many current healthcare AI systems focus on narrow applications, such as image analysis for radiology or natural language processing for clinical notes. While these specialized tools can be effective within their domains, they often fail to provide a holistic view of patient care or facilitate seamless communication across different healthcare providers and specialties. This fragmentation can lead to inefficiencies, missed opportunities for early intervention, and potential gaps in patient care.

Additionally, the vast amount of medical knowledge available, combined with the rapid pace of new research and clinical guidelines, presents a challenge for healthcare professionals to stay current and make informed decisions. Existing knowledge management systems may not adequately capture the nuances of medical information or provide context-aware recommendations based on the latest evidence.

Another limitation of current healthcare AI solutions is their reliance on structured data from electronic health records (EHRs). While EHRs contain valuable information, they often do not capture the full complexity of a patient's health status or the contextual factors that may influence treatment decisions. Furthermore, the quality and completeness of EHR data can vary significantly, potentially leading to biased or incomplete AI-driven insights.

The integration of AI technologies with existing clinical workflows also remains a challenge. Many AI tools require healthcare providers to interact with separate interfaces or systems, disrupting their normal workflow and potentially reducing adoption rates. There is a need for more seamless integration that enhances rather than complicates the healthcare delivery process.

Moreover, current systems often lack the ability to adapt to individual patient characteristics or provider preferences. Personalization in healthcare AI is still limited, with many systems providing generic recommendations that may not account for the unique circumstances of each patient or the specific expertise and experience of individual healthcare providers.

While progress has been made in developing AI-driven clinical decision support tools, many existing solutions struggle to provide clear explanations for their recommendations. This lack of transparency can lead to hesitancy among healthcare providers to rely on AI-generated insights, particularly in complex or high-stakes clinical scenarios.

Lastly, the coordination of care across multiple providers and healthcare settings remains a significant challenge. Existing systems may not effectively facilitate the sharing of relevant information or the coordination of tasks among different members of a patient's care team, potentially leading to fragmented care and suboptimal outcomes.

As healthcare continues to evolve towards more personalized, data-driven approaches, there is a growing need for integrated AI solutions that can address these limitations and provide comprehensive support for healthcare professionals and patients alike.

Several challenges persist in existing healthcare AI systems and knowledge platforms. Current solutions often operate in silos, lacking comprehensive integration across different specialties and failing to provide a holistic view of patient care. Many systems rely heavily on structured EHR data, which may not capture the full complexity of a patient's health status or contextual factors influencing treatment decisions. The integration of AI technologies with existing clinical workflows remains disruptive, potentially reducing adoption rates. Personalization capabilities are limited, with many systems providing generic recommendations that do not account for individual patient characteristics or provider preferences. There is also a lack of transparency in AI-driven clinical decision support tools, leading to hesitancy among healthcare providers to rely on their insights. Furthermore, existing platforms struggle to facilitate effective coordination of care across multiple providers and healthcare settings, potentially resulting in fragmented care and suboptimal outcomes. These unresolved issues underscore the pressing need for an integrated, multi-agent healthcare knowledge platform that can address these limitations and provide comprehensive support for both healthcare professionals and patients.

### SUMMARY

According to an aspect of the present disclosure, a method for providing health-related information to users via a healthcare knowledge platform that integrates a dynamically expandable healthcare knowledge graph, multiple specialized agents, and retrieval-augmented large language models (LLMs) is provided. The method includes receiving, by a conversational agent, a user query through a user interface; analyzing said query via a directing agent configured to determine relevant specialized agent(s) to invoke based on query type and user context and initiate sub-queries to one or more agents selected from the group consisting of a lab data agent, a visual data agent, a summarizing agent, a co-occurrence-based recommendation agent, a meta-analysis agent, or a question generation agent; retrieving data from at least one source selected from the group consisting of a large language model, a curated healthcare knowledge base, peer-reviewed journals, textbooks, or statistical co-occurrence-based data derived from electronic health records (EHRs); optionally querying the healthcare knowledge graph to determine semantic relationships among medical concepts relevant to the user query; and returning a consolidated response to the user via the conversational agent, wherein recommended expansions or additional search terms are generated through combined statistical + semantic co-occurrence analytics.

According to other aspects of the present disclosure, the method may include one or more of the following features. The method may further comprise a multi-agent subsystem comprising a conversational agent configured to interact in natural language with the user; a directing agent for routing queries to specialized agents; and one or more specialized agents selected from the group consisting of a lab data agent, a visual data agent, a summarizing agent, a co-occurrence-based recommendation agent, and a meta-analysis agent, wherein said specialized agents operate under an orchestration protocol that merges or ranks partial outputs into a coherent final result. The step of analyzing query relevance may further comprise computing semantic similarity between the retrieved data and the user query based on co-occurrence frequency metrics derived from aggregated, anonymized EHR data, and semantic relationships stored in the dynamically expandable healthcare knowledge graph, to provide context-aware results beyond purely frequency-based or purely ontology-based methods. The method may further comprise generating and maintaining the dynamically expandable healthcare knowledge graph of medical concepts and typed relationships, wherein the graph is updated in near real time based on newly published research and clinical guidelines, user or domain-expert feedback, and co-occurrence-based recommendations that merge statistical EHR data with semantic graph structures, thereby reflecting evolving clinical practices and evidence. The method may further comprise storing user queries, retrieved data, generated summaries, and co-occurrence metrics in a database, and retrieving said stored data in response to subsequent queries, wherein prior queries inform contextual or historical expansions based on similar medical codes or encounter histories. The platform may automatically generate educational question banks for healthcare trainees by analyzing the healthcare knowledge graph to identify key concepts or relationships, applying co-occurrence data to rank topics by relevance or underexplored frequency, and using an LLM for automated question generation to produce multiple-choice, short-answer, or case-based questions at varying difficulty levels. The directing agent may dynamically assign sub-queries to specialized agents based on query complexity, and subsequently merge the agent responses into a coherent, context-aware output that is displayed to the user. At least one specialized agent may be a fast-lookup agent providing a preliminary, low-latency response, while a deep-analysis agent concurrently performs a more comprehensive, retrieval-augmented query for detailed recommendations, and the directing agent may update the user interface with the deeper results when ready.

According to another aspect of the present disclosure, a system for delivering healthcare information to one or more users is provided. The system includes a user interface configured to receive queries; a multi-agent processing module having a directing agent, a conversational agent, and specialized agents for knowledge retrieval, summarization, and co-occurrence-based recommendations; a knowledge retrieval engine configured to access at least one source selected from the group consisting of large language models, curated healthcare databases, peer-reviewed journals, textbooks, or EHR-based co-occurrence data; a summarizing engine configured to generate summarized content or perform live meta-analyses of retrieved documents; a dynamically expandable healthcare knowledge graph storing medical concepts, relationships, metadata, and co-occurrence frequencies; and a database for storing user queries, retrieved data, summaries, co-occurrence metrics, and system analytics.

According to other aspects of the present disclosure, the system may include one or more of the following features. The multi-agent processing module may operate in parallel, providing an initial low-latency response while a more comprehensive analysis is performed by specialized agents that incorporate dual co-occurrence recommendations. The system may further comprise an educational module configured to automatically generate healthcare training questions tailored to user-selected topics or difficulty levels, wherein each question is linked to at least one node in the healthcare knowledge graph and optionally references co-occurrence-based expansions derived from EHR data, thereby distinguishing it from question generation systems relying solely on textual templates or simpler LLM prompts. The system may further comprise a meta-analysis module that evaluates studies based on factors selected from study quality, sample size, publication date, journal impact factor, and clinical evidence level, while also considering co-occurrence-derived expansions for broader or related search terms. The healthcare knowledge graph may be updated dynamically based on newly published research, clinical guidelines, user feedback, and combined co-occurrence data, ensuring the platform remains current with both recognized evidence and real-world usage patterns. The co-occurrence-based recommendation agent may apply a composite scoring algorithm to medical codes, prior search terms, and graph-based relationships, generating refined search recommendations that are surfaced within the multi-agent conversational interface.

According to another aspect of the present disclosure, a method for facilitating healthcare decision support is provided. The method includes receiving a real-time user query, dynamically accessing data from live clinical sources, historical EHR data, and curated medical databases, processing the query using a multi-agent system orchestrated with a dynamically updated healthcare knowledge graph, generating personalized recommendations or educational content based on combined statistical + semantic co-occurrence, and providing the results to the user through an interactive, secure interface, wherein the output includes adaptive question banks and real-time meta-analyses.

According to other aspects of the present disclosure, the method may include one or more of the following features. The method may further comprise automatically updating the healthcare knowledge graph by incorporating new research articles, clinical guidelines, and real-time clinical data, wherein machine learning algorithms continuously analyze the incoming data to adjust semantic relationships among medical entities and update co-occurrence scores. The method may further comprise generating personalized healthcare recommendations by analyzing user profiles, demographic information, and clinical history stored in the database, wherein said recommendations are integrated with the co-occurrence-based search results to yield context-specific expansions. The method may further comprise a module for real-time integration of live clinical data, wherein such data is used to update the healthcare knowledge graph and refine search or recommendation results dynamically. The method may further comprise a user feedback module that collects and analyzes user interactions and engagement metrics, wherein the feedback is employed to adapt the content of educational question banks and refine query-processing logic in the multi-agent subsystem over time. The method may further comprise detecting anomalies in co-occurrence patterns by comparing the statistical frequency of co-occurring medical codes or search terms against historical baselines, and automatically generating alerts for healthcare providers when such anomalies are detected, thereby supporting early identification of emerging trends or errors. The method may further comprise providing physician referral recommendations to a patient based on one or more input parameters, wherein the system cross-references a physician database to identify matching healthcare providers and merges that information with knowledge graph data on provider expertise. The method may further comprise automatically scheduling an appointment with a selected physician based on the patient's choice and the physician's available scheduling data, thereby creating an appointment entry in the system that is visible to the patient. The method may further comprise identifying and retrieving co-occurrence-based recommendation data by analyzing statistical co-occurrence patterns in aggregated, anonymized EHR data, analyzing semantic co-occurrence based on pathfinding or neighborhood analysis in the healthcare knowledge graph, and combining these statistical and semantic signals via a weighted scoring algorithm to generate final recommendations for the user interface. The multi-agent conversational interface may support mixed-initiative dialogue, allowing both the user and system agents to guide conversational flow, proactively suggesting relevant follow-up queries or clarifications when the knowledge graph or co-occurrence analysis indicates additional potential areas of interest. The method may further comprise generating different summarization outputs tailored to specific user roles, including concise evidence-based summaries for clinicians and simplified explanatory text for patients, wherein role-based preferences are stored in user profiles and retrieved at runtime. The method may further comprise classifying the user query involving a machine learning model trained to identify user intent and map it to one or more specialized agents in the multi-agent subsystem, thereby enabling context-driven orchestration of agent tasks.

These and other features, aspects and advantages of the present teachings will become better understood with reference to the following description, examples and appended claims.

### DRAWINGS

Those of skill in the art will understand that the drawings, described below, are for illustrative purposes only. The drawings are not intended to limit the scope of the present teachings in any way.
FIG. 1 illustrates a layered architecture of a healthcare knowledge platform, designed to integrate diverse data sources and facilitate efficient query processing.
FIG. 2 depicts a multi-agent orchestration flowchart, optimizing healthcare query processing through parallel and sequential execution paths.
FIG. 3 shows a multi-agent medical system pipeline, enabling collaborative analysis of various medical inputs for comprehensive information retrieval.
FIG. 4 presents an enhanced multi-agent medical system, incorporating laboratory analysis and information fetching for more robust medical data processing.
FIG. 5 demonstrates an automated meta-analysis process, streamlining systematic reviews through integration of advanced computational techniques with traditional methodologies.
FIG. 6 illustrates a question generation pipeline, designed to create adaptive educational content tailored to user performance and learning objectives.
FIG. 7 depicts a user role-based interface architecture, ensuring appropriate access and functionality for trainees, clinicians, and patients within the healthcare platform.
FIG. 8 shows a retrieval-augmented generation process, optimizing medical query responses by combining knowledge sources with advanced language processing techniques.

### DETAILED DESCRIPTION

All patents, applications, published applications and other publications cited herein are incorporated by reference in their entirety. Unless defined otherwise, all technical and scientific terms used herein have the same meaning as commonly understood by those of ordinary skill in the art to which the invention belongs. The chemical structures and formulae set forth herein are constructed according to the standard rules of chemical valency known in the chemical arts. Should a discrepancy exist between a depicted structure and a name given for that structure, the depicted structure is to be accorded more weight. Where the stereochemistry of a structure or a portion of a structure is not indicated in a depicted structure or a portion of the depicted structure, the depicted structure is to be interpreted as encompassing all of its possible stereoisomers.

Any methods, devices and materials similar or equivalent to those described herein can be used in the practice of this invention. The following definitions are provided to facilitate understanding of certain terms used frequently herein and are not meant to limit the scope of the present disclosure. In the event that there is a plurality of definitions for a term herein, those in this section prevail unless stated otherwise. Headings used herein are for organizational purposes only and in no way limit the invention described herein.

### Abbreviations and Definitions

AI: As used herein, the term "AI" refers to artificial intelligence, which encompasses computer systems and algorithms designed to perform tasks that typically require human intelligence, such as visual perception, speech recognition, decision-making, and language translation.

EHR: As used herein, the term "EHR" refers to Electronic Health Record, a digital version of a patient's paper chart that contains real-time, patient-centered records and makes information available instantly and securely to authorized users.

LLM: As used herein, the term "LLM" refers to Large Language Model, an advanced artificial intelligence model trained on vast amounts of text data, capable of understanding, generating, and manipulating human-like text based on input prompts.

KG: As used herein, the term "KG" refers to Knowledge Graph, a structured representation of information in the form of entities and relationships between them, used to store and organize complex medical knowledge.

RAG: As used herein, the term "RAG" refers to Retrieval-Augmented Generation, a technique that combines information retrieval from external sources with language generation to produce more accurate and contextually relevant responses.

NLP: As used herein, the term "NLP" refers to Natural Language Processing, a branch of artificial intelligence that focuses on the interaction between computers and humans using natural language, enabling machines to understand, interpret, and generate human language.

FHIR: As used herein, the term "FHIR" refers to Fast Healthcare Interoperability Resources, a standard for exchanging healthcare information electronically, designed to facilitate interoperability between legacy health care systems.

API: As used herein, the term "API" refers to Application Programming Interface, a set of protocols, routines, and tools for building software applications that specifies how software components should interact.

HIPAA: As used herein, the term "HIPAA" refers to the Health Insurance Portability and Accountability Act, a US law designed to provide privacy standards to protect patients' medical records and other health information provided to health plans, doctors, hospitals, and other healthcare providers.

GDPR: As used herein, the term "GDPR" refers to the General Data Protection Regulation, a regulation in EU law on data protection and privacy for all individuals within the European Union and the European Economic Area.

### Multi-Agent Healthcare Knowledge Platform with Co-Occurrence-Driven Intelligence

The present invention relates to systems and methods for providing comprehensive healthcare information and support through an integrated, multi-agent platform. This platform leverages advanced artificial intelligence techniques, including large language models and dynamic knowledge graphs, to deliver contextually relevant medical information, clinical decision support, and interactive educational resources.

In some cases, the invention may address limitations of existing healthcare information systems by uniquely combining multiple specialized agents, a dynamically expandable medical knowledge graph, and sophisticated co-occurrence analysis derived from both electronic health records and semantic relationships. This approach may enable more accurate, context-aware, and personalized healthcare guidance than conventional single-agent or rule-based systems.

The platform may incorporate retrieval-augmented generation techniques to ground responses in current medical literature and clinical guidelines, potentially reducing the risk of outdated or inaccurate information. In some cases, the system may dynamically update its knowledge base through automated processing of new research, clinical data, and user feedback, allowing it to evolve with advancing medical knowledge.

In some implementations, the invention may provide end-to-end support for healthcare workflows, from initial patient interaction through diagnosis, treatment recommendations, and follow-up care. This may include capabilities for automated physician referrals and appointment scheduling, addressing logistical challenges in healthcare delivery.

The multi-agent architecture of the system may allow for specialized processing of different types of medical data and queries, potentially improving accuracy and relevance compared to single-model approaches. In some cases, the platform may generate adaptive educational content and assessments, tailoring learning experiences to individual users based on their role, knowledge level, and performance.

By integrating these diverse capabilities into a unified platform, the invention may represent a novel approach to healthcare knowledge management and dissemination. The synergistic combination of advanced AI techniques, dynamic knowledge representation, and workflow integration may provide unexpected utility in addressing complex healthcare information needs across multiple user types, including clinicians, patients, and medical trainees.

Those skilled in the art will recognize that various modifications and variations can be made without departing from the spirit and scope of the invention.

### System Architecture

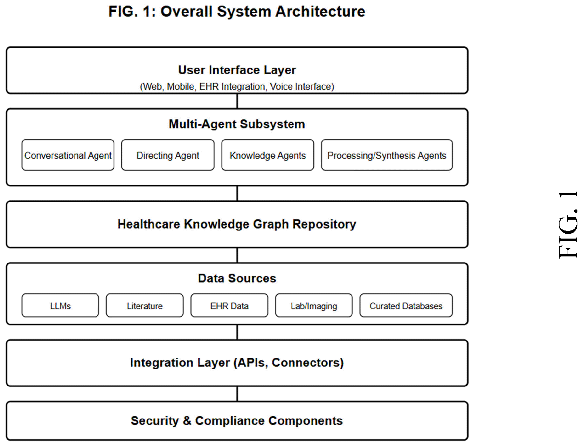

The healthcare knowledge platform may comprise a layered architecture as illustrated in FIG. 1. This architecture may include multiple interconnected layers that work together to process healthcare queries and provide relevant information to users.

In some cases, the topmost layer of the architecture may be a User Interface Layer. This layer may provide various access points for users to interact with the system. The User Interface Layer may include web applications compatible with different browsers, allowing users to access the platform through standard web interfaces. In some implementations, native mobile applications for iOS and Android platforms may be developed to provide optimized experiences on smartphones and tablets. Desktop applications may also be created for users who prefer working on personal computers or workstations.

The User Interface Layer may additionally support plugins or embedded modules that can be integrated directly into existing Electronic Health Record (EHR) systems. This integration may allow healthcare providers to access the platform's capabilities without leaving their primary clinical software environment. For developers and third-party services, Application Programming Interfaces (APIs) may be made available, enabling programmatic access to the platform's functionalities and data.

Input modalities supported by the User Interface Layer may vary. In some cases, text-based chat interfaces may be implemented, allowing users to type natural language queries. Voice command interfaces utilizing speech-to-text engines may be incorporated, potentially with fine-tuning for medical terminology to improve accuracy. Structured data entry forms may be provided for specific types of queries, such as patient intake information or clinical trial search parameters. The system may also support image upload capabilities, enabling visual queries like "Identify conditions related to this rash image."

Below the User Interface Layer, FIG. 1 depicts a Multi-Agent Subsystem. This layer may contain various specialized agents that work in concert to process user queries and generate responses. The Multi-Agent Subsystem may include a Conversational Agent for handling natural language interactions, a Directing Agent for orchestrating query processing across other agents, and multiple Knowledge Agents and Processing/Synthesis Agents for retrieving and analyzing information from various sources.

At the core of the architecture, a Healthcare Knowledge Graph Repository may serve as the semantic foundation of the system. This layer may store and manage the dynamic healthcare knowledge graph, encoding medical concepts, relationships, and metadata.

The Data Sources layer illustrated in FIG. 1 may encompass connections to diverse information repositories. These may include Large Language Models (LLMs) for advanced natural language processing, literature databases for accessing peer-reviewed medical research, Electronic Health Record (EHR) data for real-world clinical insights, laboratory and imaging systems for diagnostic information, and curated databases containing validated medical knowledge.

An Integration Layer may be implemented to manage APIs and connectors to external systems. This layer may facilitate secure and standardized communication between the platform and other healthcare IT systems, ensuring interoperability and data exchange capabilities.

The bottom layer of the architecture in FIG. 1 shows Security & Compliance Components. These components may be responsible for ensuring data protection, user authentication, access control, and adherence to healthcare regulations such as HIPAA or GDPR.

In terms of deployment options, the healthcare knowledge platform may be implemented in various configurations to suit different organizational needs and infrastructure requirements. In some cases, the system may be deployed as a fully cloud-based Software-as-a-Service (SaaS) platform. This approach may allow users to access the platform through standard web browsers or mobile applications, with all processing and data storage handled in secure cloud environments.

Alternatively, the platform may be installed on-premise within a healthcare institution's own data center. This deployment model may provide maximum control over sensitive data and may be preferred by organizations with strict data residency requirements or existing investments in local infrastructure.

A hybrid deployment model may also be possible, where certain components of the system (such as those handling sensitive patient data) are deployed on-premise, while other components (like general knowledge processing or user interfaces) operate in the cloud. This approach may balance the benefits of cloud scalability with the security advantages of local data control.

Those skilled in the art will recognize that various modifications and variations can be made to the system architecture without departing from the spirit and scope of the invention. For example, additional layers or components may be added to address specific organizational needs or to incorporate emerging technologies as they become available.

### Multi-Agent Subsystem

The healthcare knowledge platform may incorporate a multi-agent subsystem to process user queries and generate responses. This subsystem may comprise various specialized agents working in concert to analyze, retrieve, and synthesize medical information.

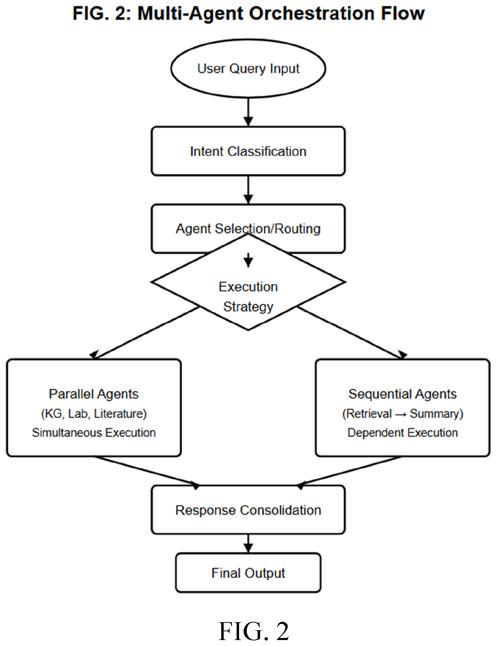

As illustrated in FIG. 2, the multi-agent orchestration flow may begin with User Query Input. The query may then undergo Intent Classification to determine the nature of the user's request. Based on this classification, the system may proceed to Agent Selection/Routing, where appropriate specialized agents are chosen to handle the query.

FIG. 2 depicts an Execution Strategy decision point, which may lead to either Parallel Agents or Sequential Agents processing. In the Parallel Agents path, Knowledge Graph (KG), Lab, and Literature queries may be executed simultaneously. The Sequential Agents path may involve a Retrieval step followed by a Summary step in a dependent sequence. Both paths may converge at a Response Consolidation stage, where results are merged into a coherent Final Output for the user.

In some cases, the multi-agent subsystem may be configured with different subsets of specialized agents to suit various use cases or deployment scenarios. A minimal core embodiment may comprise a Conversational Agent, Directing Agent, Knowledge Graph Query Agent, a basic Literature Retrieval Agent, and a Large Language Model (LLM) Agent with retrieval-augmented generation capabilities.

Expanded versions of the multi-agent subsystem may be tailored for specific focuses. For clinical decision support, additional agents such as a Lab Data Agent, Imaging Data Agent, Electronic Health Record (EHR) Data Agent, Co-occurrence Agent, advanced Summarization Agent, and Meta-Analysis Agent may be incorporated. An education-focused configuration may emphasize a Question Generation Agent and potentially a Simulation Agent, integrating with curriculum-specific knowledge graph nodes and textbook data.

For patient navigation purposes, the system may prioritize a Physician Referral Agent and an Appointment Scheduling Agent, potentially with simplified language generation via the LLM Agent. A research-oriented embodiment may feature advanced Meta-Analysis and Co-occurrence agents, potentially a Clinical Trial Matching agent, and robust API access for data extraction and analysis.

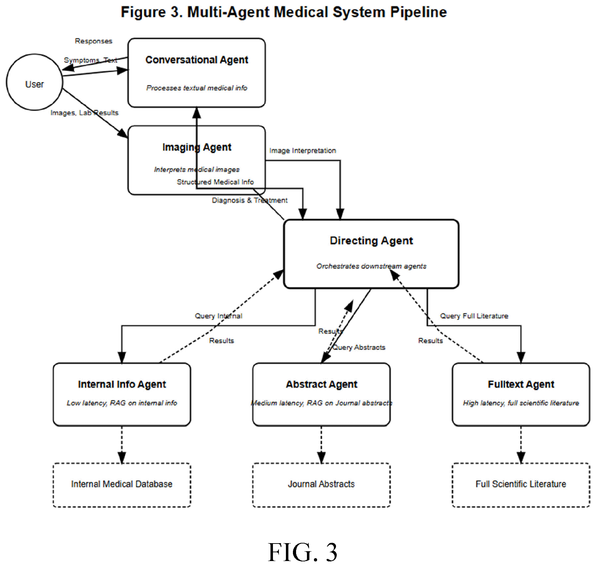

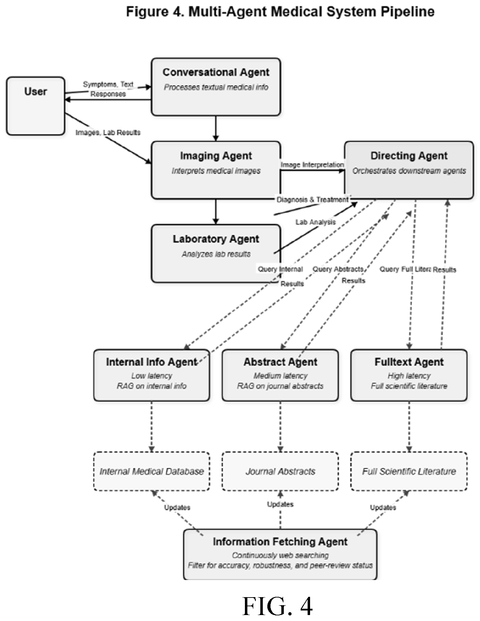

FIG. 3 and FIG. 4 illustrate potential configurations of the multi-agent medical system pipeline. In these figures, a User interacts with a Conversational Agent by providing symptoms, textual information, images, and lab results. The Conversational Agent may process textual medical information, while specialized agents handle other input types. For example, an Imaging Agent may interpret medical images and provide structured medical information along with diagnosis and treatment recommendations.

As shown in FIG. 3 and FIG. 4, a central Directing Agent may orchestrate downstream information retrieval by coordinating with specialized knowledge agents. These may include an Internal Info Agent for low-latency retrieval from internal databases, an Abstract Agent for medium-latency retrieval from journal abstracts, and a Fulltext Agent for high-latency retrieval from full scientific literature.

FIG. 4 additionally depicts a Laboratory Agent that may analyze lab results and provide structured data to the Directing Agent. An Information Fetching Agent may receive updates from various knowledge repositories, filter and process this information before updating the knowledge bases.

Individual agents within the multi-agent subsystem may be implemented using various underlying technologies. The Conversational Agent, for instance, may utilize different Natural Language Understanding (NLU) or Natural Language Processing (NLP) models. These models may be based on transformer architectures or other advanced language processing techniques, potentially fine-tuned on medical conversations to improve domain-specific performance.

The LLM Agent may leverage various large language models, which may differ in size, training data, or fine-tuning approaches. In some cases, the system may employ multiple LLMs, selecting the most appropriate model based on the specific task or query type.

The Directing Agent, responsible for orchestrating query processing across other agents, may be implemented using rule-based systems, machine learning models, or hybrid approaches. Machine learning models used for agent selection and routing may be trained on historical query data to improve decision-making over time.

Specialized agents like the Co-occurrence Agent or Meta-Analysis Agent may employ various statistical methods or machine learning models tailored to their specific functions. For example, the Co-occurrence Agent may use techniques ranging from simple frequency analysis to more sophisticated machine learning approaches for identifying meaningful associations in medical data.

Those skilled in the art will recognize that various modifications and variations can be made to the multi-agent subsystem without departing from the spirit and scope of the invention. The specific configuration and implementation of agents may be adapted based on the intended use case, available computational resources, and evolving artificial intelligence technologies.

### Dynamic Healthcare Knowledge Graph

The healthcare knowledge platform may incorporate a dynamic healthcare knowledge graph as a core component of its architecture. This knowledge graph may serve as a structured representation of medical information, encoding complex relationships between various healthcare concepts.

In some cases, the knowledge graph may comprise nodes representing diverse medical entities. These nodes may include diseases, symptoms, procedures, drugs, anatomical structures, genes, proteins, biomarkers, laboratory tests, microorganisms, clinical trials, guidelines, publications, healthcare providers, and abstract medical concepts. The relationships between these entities may be represented by typed edges in the graph structure.

The edges in the knowledge graph may encode various types of relationships. For example, edges may represent treatment relationships between drugs and diseases, causal relationships between conditions, diagnostic relationships between symptoms and disorders, or hierarchical relationships within anatomical structures. In some implementations, edges may also capture contraindications, drug interactions, or associations between healthcare providers and their areas of expertise.

Nodes and edges within the knowledge graph may store additional properties and metadata. This metadata may include definitions, synonyms, standardized medical codes (such as ICD, SNOMED CT, LOINC, or RxNorm), quantitative data like drug dosage ranges or laboratory reference values, and provenance information indicating the source of the data. In some cases, confidence scores may be associated with relationships, reflecting the strength of evidence supporting a particular connection.

The dynamic nature of the healthcare knowledge graph may allow for continuous updates and expansion of its content. In some implementations, updates to the knowledge graph may occur in real-time as new information becomes available. For example, newly published research articles or clinical guidelines may be automatically processed and incorporated into the graph structure shortly after publication.

Alternatively, the knowledge graph may be updated through scheduled batch processes. These batch updates may involve periodic ingestion of data from various sources, potentially including literature databases, clinical trial registries, or curated medical knowledge bases. The frequency of these batch updates may be adjusted based on the specific needs of the healthcare organization or the rate of change in medical knowledge within particular domains.

In some cases, a semi-automated update process may be employed. This approach may involve automated extraction of information from new sources, followed by human review and validation before incorporation into the live knowledge graph. This method may help maintain the accuracy and reliability of the graph while still allowing for regular expansion of its content.

Manual curation may also play a role in maintaining and expanding the knowledge graph. Domain experts may directly add, modify, or remove relationships within the graph based on their specialized knowledge or in response to emerging medical consensus. This manual curation process may be particularly valuable for incorporating nuanced clinical insights that may be challenging to extract automatically from published literature.

The scope of the healthcare knowledge graph may vary depending on the specific implementation and use case. In some cases, the knowledge graph may focus on one or more specific medical specialties. For example, a knowledge graph tailored for oncology may include detailed information about cancer types, staging systems, treatment protocols, and relevant genetic markers. This specialized approach may allow for greater depth of coverage within a particular domain.

Alternatively, the knowledge graph may be designed to encompass all of medicine. This comprehensive approach may facilitate cross-specialty insights and support a wider range of healthcare queries. However, maintaining a broad-scope knowledge graph may require more extensive computational resources and curation efforts.

The healthcare knowledge graph may be implemented using various graph database technologies. In some cases, native graph databases such as Neo4j or ArangoDB may be employed. These databases are optimized for storing and querying graph structures, potentially offering performance benefits for complex relationship traversals.

Cloud-based graph database services like Amazon Neptune may be utilized in some implementations. These services may provide scalability and managed infrastructure, potentially reducing the operational overhead of maintaining the knowledge graph.

In other cases, RDF triple stores may be used to implement the knowledge graph. This approach may offer advantages in terms of standardization and interoperability, particularly when working with existing medical ontologies or linked open data resources.

The choice of graph database technology may depend on factors such as the expected scale of the knowledge graph, query performance requirements, integration needs with other components of the healthcare platform, and the specific types of analyses to be performed on the graph data.

The healthcare knowledge graph may play a crucial role in providing context for other components of the system. For example, when processing natural language queries, entities and relationships identified in the query may be mapped to corresponding nodes and edges in the knowledge graph. This mapping may allow for more precise interpretation of user intent and expansion of the query to related concepts.

In some implementations, the knowledge graph may be used to ground the outputs of large language models, ensuring that generated text aligns with established medical knowledge. The graph structure may also inform the generation of educational content, helping to identify key concepts and relationships that should be covered in training materials or assessment questions.

The co-occurrence analysis capabilities of the system may be enhanced by the knowledge graph. Statistical co-occurrence patterns derived from electronic health records may be combined with semantic relationships encoded in the graph to generate more meaningful and clinically relevant recommendations.

Those skilled in the art will recognize that various modifications and variations can be made to the structure and implementation of the healthcare knowledge graph without departing from the spirit and scope of the invention. The specific technologies, update mechanisms, and scope of coverage may be adapted based on the intended use case, available resources, and evolving standards in medical informatics and graph database technologies.

### Information Retrieval and Synthesis

The healthcare knowledge platform may employ sophisticated information retrieval and synthesis techniques to provide accurate and contextually relevant responses to user queries. This process may involve accessing and integrating data from multiple sources, including large language models (LLMs), curated databases, peer-reviewed literature, and electronic health record (EHR) data.

In some cases, the system may integrate different combinations of data sources to suit specific deployment scenarios or organizational needs. For example, one implementation may rely solely on publicly available literature sources such as PubMed Central or open-access journals. This approach may be suitable for educational or research-focused applications where broad coverage of published medical knowledge is prioritized.

Alternatively, the platform may incorporate licensed commercial databases containing specialized medical information. These may include drug compendia, clinical practice guidelines from professional societies, or proprietary collections of curated medical knowledge. Access to such resources may enhance the depth and specificity of information available to the system, potentially improving its utility for clinical decision support applications.

In some implementations, the platform may integrate proprietary institutional data. This may include internal protocols, anonymized patient outcome data, or locally developed clinical pathways. Incorporation of such data may allow the system to provide recommendations that are more closely aligned with the practices and experiences of a specific healthcare organization.

The healthcare knowledge platform may also leverage real-time data feeds in some cases. These may include live laboratory results, vital sign monitoring data, or updates from connected medical devices. Integration of real-time data may enable the system to provide more timely and personalized responses, particularly in acute care or monitoring scenarios.

Some implementations of the platform may incorporate curated web data from reputable medical websites. This approach may require careful validation and source attribution processes to ensure the reliability of the information. Web data integration may be particularly useful for capturing rapidly evolving medical knowledge or public health information that has not yet been formally published in peer-reviewed literature.

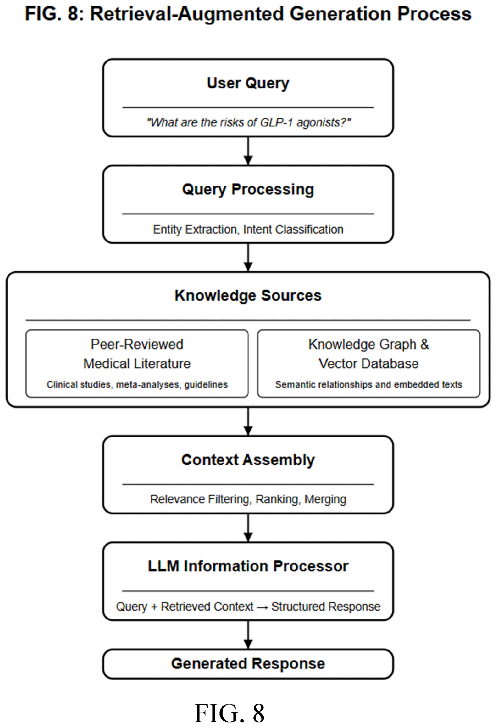

The retrieval process employed by the healthcare knowledge platform may vary in sophistication depending on the specific implementation and use case. As illustrated in FIG. 8, the process may begin with a user query, which undergoes query processing to extract relevant entities and classify the intent of the request.

In some cases, the system may employ keyword-based search techniques as part of its retrieval strategy. This approach may involve matching terms from the processed query against indexed documents or database fields. Keyword search may be particularly effective for finding exact matches or when dealing with well-structured, domain-specific terminology.

Alternatively, or in addition to keyword search, the platform may utilize semantic vector search techniques. This approach may involve converting the query and potential results into high-dimensional vector representations that capture semantic meaning. Vector similarity calculations may then be used to identify the most relevant information, potentially surfacing conceptually related content even when exact keyword matches are not present.

In some implementations, a hybrid approach combining both keyword and semantic search may be employed. This strategy may leverage the strengths of both methods, using keyword matching for precision and semantic search for recall and conceptual expansion.

The healthcare knowledge platform may employ varying levels of sophistication in its Retrieval-Augmented Generation (RAG) process. As depicted in FIG. 8, this process may involve assembling relevant context from knowledge sources such as peer-reviewed medical literature and a combined knowledge graph and vector database. The retrieved context may then be used to inform and ground the outputs of a large language model, ensuring that generated responses are based on current and accurate medical information.

In some cases, the RAG process may involve simple concatenation of retrieved text snippets with the user query before passing to the LLM. More advanced implementations may employ techniques such as re-ranking retrieved passages based on relevance to the specific query, or dynamically adjusting the amount of context provided based on the complexity of the question.

The platform may also implement sophisticated filtering and merging strategies during the context assembly phase. This may involve removing redundant information, resolving conflicts between different sources, or prioritizing more recent or authoritative content. In some implementations, the system may maintain provenance information throughout the retrieval and generation process, allowing for clear attribution of sources in the final output.

The synthesis of information from multiple sources may present challenges in terms of consistency and coherence. To address this, the healthcare knowledge platform may employ various techniques for harmonizing and integrating diverse data. In some cases, this may involve mapping concepts from different sources to a common ontology or terminology system. The platform may also utilize natural language generation techniques to produce fluent, unified responses that seamlessly incorporate information from multiple sources.

Those skilled in the art will recognize that various modifications and variations can be made to the information retrieval and synthesis processes without departing from the spirit and scope of the invention. The specific combination of data sources, retrieval strategies, and synthesis techniques may be adapted based on the intended use case, available computational resources, and evolving standards in medical informatics and natural language processing.

### Meta-Analysis and Systematic Review

The healthcare knowledge platform may incorporate advanced capabilities for automated meta-analysis and systematic review of medical literature. This functionality may enable users to rapidly synthesize evidence from multiple studies, potentially supporting more informed clinical decision-making and research activities.

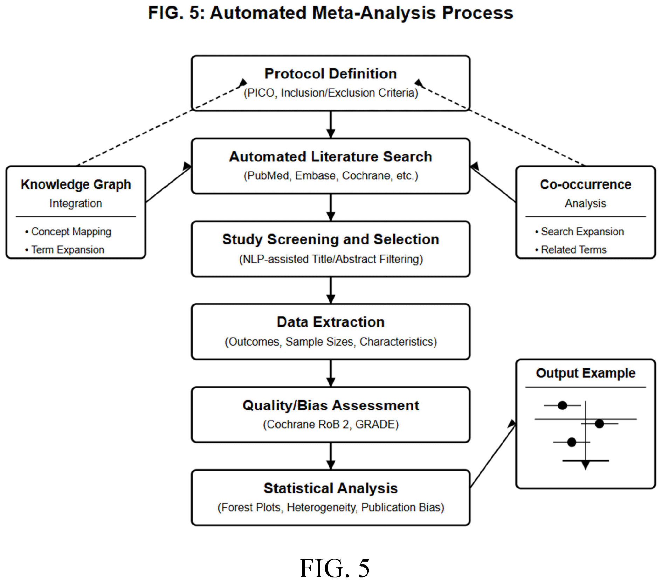

As illustrated in FIG. 5, the automated meta-analysis process may begin with Protocol Definition. In some cases, this step may involve specifying PICO (Population, Intervention, Comparison, Outcome) elements and defining inclusion/exclusion criteria for studies to be considered. The system may allow users to input these parameters through structured forms or natural language interfaces.

Following protocol definition, the process may proceed to Automated Literature Search. The system may execute comprehensive searches across multiple literature databases such as PubMed, Embase, and Cochrane Library. In some implementations, the search strategy may be informed by Knowledge Graph Integration, which may provide concept mapping and term expansion capabilities. This integration may help ensure that relevant synonyms and related concepts are included in the search, potentially improving the comprehensiveness of the literature retrieval.

The automated meta-analysis process may also leverage Co-occurrence Analysis to enhance the search strategy. As shown in FIG. 5, this analysis may contribute to search expansion and suggestion of related terms. By incorporating co-occurrence data derived from electronic health records and semantic relationships in the knowledge graph, the system may identify clinically relevant associations that might not be immediately apparent from the initial search terms alone.

Once relevant literature has been identified, the process may move to Study Screening and Selection. In some cases, this step may involve Natural Language Processing (NLP) assisted Title/Abstract Filtering. Machine learning models trained on medical text may be employed to rapidly screen large numbers of abstracts, potentially reducing the manual workload for researchers. The system may flag studies that meet the predefined inclusion criteria for further review.

Data Extraction may follow the screening process. The system may attempt to automatically extract key information from selected studies, including outcomes, sample sizes, and study characteristics. In some implementations, this extraction may be performed using specialized NLP models designed to identify and structure relevant data from full-text articles. The extracted data may be presented in a standardized format for review and potential manual correction if needed.

Quality and Bias Assessment may be an integral part of the meta-analysis process. As depicted in FIG. 5, the system may incorporate tools such as Cochrane RoB 2 (Risk of Bias 2) for randomized controlled trials or ROBINS-I for non-randomized studies. These assessments may help evaluate the methodological rigor of included studies and inform the interpretation of results. In some cases, the system may also apply the GRADE (Grading of Recommendations, Assessment, Development and Evaluations) methodology to assess the overall quality of evidence for each outcome.

The final stage of the process may involve Statistical Analysis. The system may implement various meta-analytical techniques depending on the nature of the data and research question. In some cases, this may include generating Forest Plots to visually represent effect sizes and confidence intervals across studies. The system may compute measures of heterogeneity, such as I² statistics, to assess the consistency of results across studies. Publication bias may be evaluated through the creation of funnel plots and statistical tests such as Egger's regression or Begg's rank correlation test.

The range of meta-analysis capabilities offered by the system may vary depending on the specific implementation and use case. In some cases, the platform may provide rapid evidence summaries based on existing systematic reviews and meta-analyses. This approach may be suitable for quick clinical decision support, offering an overview of current evidence without the need for a full de novo analysis.

At the other end of the spectrum, the system may support comprehensive, de novo statistical meta-analyses. In these cases, users may have more control over the analytical process, potentially specifying models (e.g., fixed-effect vs. random-effects), effect measures (e.g., odds ratios, risk ratios, mean differences), and advanced techniques such as meta-regression or subgroup analyses.

The output of the meta-analysis process may be tailored to different user needs. For clinicians, the system may generate concise summaries highlighting key findings and their implications for practice. Researchers may receive more detailed reports including full statistical outputs, sensitivity analyses, and suggestions for future research directions. In some implementations, the system may produce draft manuscripts following standard reporting guidelines such as PRISMA (Preferred Reporting Items for Systematic Reviews and Meta-Analyses).

Interactive visualizations may be generated to allow users to explore the meta-analysis results. These may include dynamic forest plots where users can adjust study weights or exclude studies to see the impact on overall effect estimates. In some cases, the system may provide tools for real-time updating of meta-analyses as new studies become available, potentially supporting living systematic reviews.

The automated meta-analysis and systematic review capabilities of the healthcare knowledge platform may be integrated with other components of the system. For example, results from meta-analyses may be incorporated into the dynamic healthcare knowledge graph, potentially informing future query responses or clinical decision support recommendations. The co-occurrence analysis used to enhance literature searches may also benefit from the synthesized evidence, creating a feedback loop that continually refines the system's knowledge base.

Those skilled in the art will recognize that various modifications and variations can be made to the meta-analysis and systematic review processes without departing from the spirit and scope of the invention. The specific statistical methods, quality assessment tools, and output formats may be adapted based on evolving best practices in evidence synthesis, computational capabilities, and the needs of different user groups within the healthcare ecosystem.

### Educational Content Generation

The healthcare knowledge platform may incorporate advanced capabilities for generating educational content, particularly in the form of adaptive question banks tailored to various user roles and knowledge levels. This functionality may support continuous learning and assessment for healthcare professionals, trainees, and potentially patients.

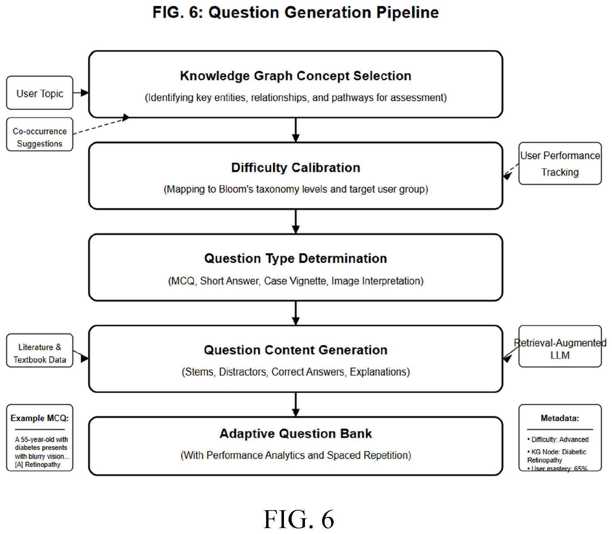

As illustrated in FIG. 6, the question generation pipeline may begin with Knowledge Graph Concept Selection. In some cases, this step may involve identifying key entities, relationships, and pathways within the healthcare knowledge graph that are relevant to the desired educational content. The selection process may be influenced by User Topic inputs, allowing learners or educators to specify areas of focus. Additionally, Co-occurrence Suggestions derived from analysis of electronic health record data and semantic relationships in the knowledge graph may inform the concept selection, potentially highlighting clinically relevant associations or frequently encountered scenarios.

Following concept selection, the process may proceed to Difficulty Calibration. This step may involve mapping content to appropriate levels of complexity, potentially using frameworks such as Bloom's taxonomy to ensure a range of cognitive skills are addressed. In some implementations, the difficulty calibration may be informed by User Performance Tracking data, allowing the system to adapt question difficulty based on individual learner progress and areas of strength or weakness.

The pipeline may then move to Question Type Determination. The system may generate various formats of questions, which may include but are not limited to Multiple Choice Questions (MCQs), Short Answer questions, Case Vignettes, and Image Interpretation tasks. The selection of question types may be influenced by the nature of the content, the intended learning objectives, and potentially user preferences or learning style data.

Question Content Generation may follow, involving the creation of question stems, distractors (for MCQs), correct answers, and explanations. This stage may utilize input from Literature & Textbook Data to ensure accuracy and relevance of the content. In some cases, Retrieval-Augmented LLM assistance may be employed to aid in the generation process, potentially improving the quality and diversity of generated questions.

The output of the pipeline may be an Adaptive Question Bank that incorporates Performance Analytics and Spaced Repetition features. This adaptive system may continually adjust the presentation of questions based on learner performance, potentially prioritizing areas that require reinforcement while spacing out well-understood concepts to optimize long-term retention.

In some implementations, the question generation capabilities may span a wide range of complexity. At the simpler end, the system may produce questions focused on fact recall, directly testing knowledge of specific medical concepts, definitions, or relationships encoded in the knowledge graph. For example, a basic question might ask about the primary symptoms associated with a particular condition or the mechanism of action of a specific medication.

At the more advanced end of the spectrum, the system may generate complex, adaptive case vignettes that require clinical reasoning and decision-making skills. These vignettes may present detailed patient scenarios, potentially incorporating multiple comorbidities, laboratory results, and imaging findings. Learners may be asked to navigate through the case, making diagnostic and treatment decisions at various points. The system may adapt the progression of the case based on the learner's choices, simulating the complexities of real-world clinical practice.

In some cases, the question generation process may incorporate elements of gamification or simulation. For example, the system may create interactive scenarios where learners can virtually examine patients, order and interpret diagnostic tests, and develop treatment plans. These simulations may be particularly valuable for training in areas such as emergency medicine or surgical decision-making, where rapid assessment and action are critical.

The adaptive nature of the question bank may extend beyond individual questions to encompass entire learning paths. The system may analyze patterns in user performance across multiple topics and question types to identify knowledge gaps or areas of particular strength. Based on this analysis, the platform may suggest personalized learning objectives or curate sets of questions designed to address specific developmental needs.

In some implementations, the question generation system may incorporate collaborative or social learning features. For example, educators may be able to review and curate automatically generated questions, potentially adding their own insights or modifying content to align with specific curriculum requirements. Learners may have the option to engage in peer review of responses to open-ended questions, fostering critical thinking and discussion.

The platform may also support the creation of specialized question sets for particular use cases. For instance, it may generate practice questions mimicking the format and content of specific medical licensing exams or board certifications. Alternatively, it may produce question banks tailored to patient education, using simplified language and focusing on practical self-management strategies for chronic conditions.

To ensure the ongoing relevance and accuracy of generated questions, the system may implement feedback loops and quality control mechanisms. In some cases, this may involve periodic review of question performance metrics, flagging items that consistently perform poorly for expert review. The system may also incorporate mechanisms for users to report potential errors or suggest improvements, allowing for continuous refinement of the question bank.

Those skilled in the art will recognize that various modifications and variations can be made to the educational content generation process without departing from the spirit and scope of the invention. The specific algorithms for question generation, adaptive learning, and performance analysis may be tailored to suit different educational philosophies, medical specialties, or technological capabilities.

### User Role-Based Interfaces

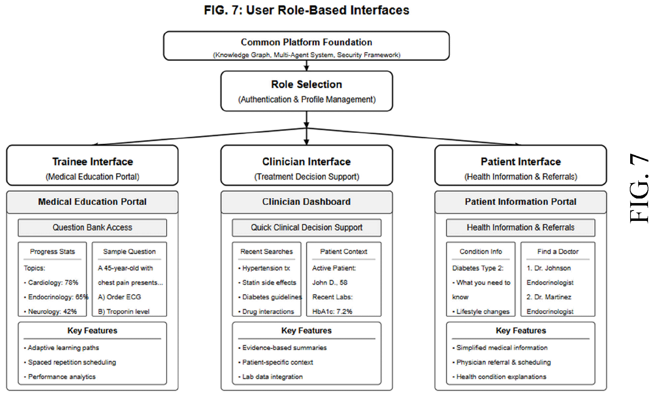

The healthcare knowledge platform may implement user role-based interfaces to provide tailored access and functionality for different types of users. As illustrated in FIG. 7, the system may offer distinct interface branches for trainees, clinicians, and patients, each designed to meet the specific needs and workflows of these user groups.

In some cases, the platform may employ a common foundation that underpins all user interfaces. This shared infrastructure may include core components such as the healthcare knowledge graph, retrieval and synthesis engines, and security protocols. The role-based interfaces may then build upon this foundation, presenting information and tools in ways that are most relevant and accessible to each user type.

For trainees, the interface may focus on educational content and assessment tools. In some implementations, this may include access to adaptive question banks, performance tracking dashboards, and simulated case studies. The trainee interface may integrate closely with the platform's question generation capabilities, potentially allowing users to create custom study plans or practice exams tailored to their specific learning objectives or upcoming assessments.

The clinician interface may prioritize clinical decision support and patient data integration. In some cases, this may involve presenting summarized evidence from meta-analyses alongside individual patient data retrieved from electronic health records. The interface may offer rapid access to treatment guidelines, drug interaction checkers, and diagnostic support tools. In some implementations, the clinician interface may also provide capabilities for documenting clinical reasoning or generating structured notes based on interactions with the platform.

For patients, the interface may emphasize accessible health information, self-management tools, and care navigation support. In some cases, this may include simplified explanations of medical concepts, personalized health tracking features, and interfaces for managing appointments or medication schedules. The patient interface may also incorporate the platform's physician referral and appointment scheduling capabilities, potentially streamlining the process of connecting patients with appropriate healthcare providers.

Each role-based interface may implement adaptive presentation logic to further tailor the user experience. In some implementations, this may involve tracking user behavior and preferences over time, adjusting the prominence of different features or the complexity of presented information based on observed usage patterns or explicitly set preferences.

The system may also support customization of interfaces at an organizational level. In some cases, healthcare institutions may be able to configure role-based interfaces to align with specific clinical pathways, institutional protocols, or local regulations. This customization may extend to branding elements, terminology preferences, or the integration of institution-specific resources and tools.

In some implementations, the platform may support dynamic role switching or multi-role access. For example, a user who is both a clinician and an educator may be able to seamlessly transition between interfaces optimized for patient care and those designed for managing trainee assessments. The system may maintain appropriate access controls and data segregation to ensure that sensitive information is only accessible within the appropriate context.

The role-based interfaces may also incorporate collaborative features that span user types. In some cases, this may include shared workspaces where clinicians and trainees can review cases together, or secure messaging systems that facilitate communication between patients and their care teams. These collaborative tools may be designed to respect role boundaries while promoting effective information sharing and teamwork.

Accessibility considerations may be integrated into the design of all role-based interfaces. In some implementations, this may involve support for screen readers, keyboard navigation, and customizable color schemes to accommodate users with visual impairments. The system may also offer multilingual support, potentially leveraging the platform's language processing capabilities to provide dynamic translation of interface elements and content.

Mobile responsiveness may be a key feature of the role-based interfaces. In some cases, the system may offer native mobile applications tailored to each user role, optimizing the interface for smaller screens and touch-based interactions. These mobile interfaces may prioritize quick access to frequently used features and may incorporate device-specific capabilities such as biometric authentication for secure access.

The platform may implement robust analytics and feedback mechanisms across all role-based interfaces. In some implementations, this may involve tracking user engagement metrics, collecting explicit feedback through surveys or rating systems, and analyzing usage patterns to identify areas for improvement. This data may be used to continuously refine and evolve the interfaces, ensuring they remain aligned with user needs and emerging best practices in healthcare information delivery.

Those skilled in the art will recognize that various modifications and variations can be made to the user role-based interfaces without departing from the spirit and scope of the invention. The specific features, layouts, and interaction models may be adapted based on evolving user needs, technological capabilities, and healthcare delivery paradigms.

### Data Storage and Retrieval

The healthcare knowledge platform may employ various data storage and retrieval mechanisms to efficiently manage and access the large volumes of information required for its operation. In some cases, the system may utilize a combination of specialized database technologies, each optimized for specific types of data and query patterns.

For storing and querying the dynamic healthcare knowledge graph, the platform may employ a graph database such as Neo4j, Neptune, or ArangoDB. Graph databases may be particularly well-suited for representing and traversing complex relationships between medical entities. In some implementations, the system may utilize distributed graph processing frameworks to handle very large-scale knowledge graphs spanning multiple servers or cloud instances.

To support efficient similarity search for retrieval-augmented generation and semantic querying, the platform may incorporate a vector database such as Pinecone, Milvus, or Weaviate. Vector databases may enable rapid nearest-neighbor lookups in high-dimensional spaces, potentially improving the speed and relevance of information retrieval operations.

For storing and indexing unstructured or semi-structured data such as medical literature, clinical notes, or user interaction logs, the system may employ document-oriented databases like MongoDB or Elasticsearch. These databases may offer flexible schema designs and powerful full-text search capabilities, potentially facilitating dynamic updates to the knowledge base and efficient content retrieval.

Structured data such as user profiles, question banks, or standardized medical codes may be stored in relational databases like PostgreSQL or MySQL. Relational databases may provide robust support for complex queries, transactions, and data integrity constraints, which may be valuable for managing core operational data of the platform.

In some cases, the platform may implement a polyglot persistence architecture, using multiple database technologies in combination to leverage the strengths of each for different data types and access patterns. This approach may involve careful design of data models and synchronization mechanisms to maintain consistency across different storage systems.

The healthcare knowledge platform may implement various strategies to ensure data privacy and security. In some cases, this may involve end-to-end encryption for data in transit and at rest, potentially using strong encryption algorithms such as AES-256. The system may employ granular access controls based on user roles and data sensitivity levels, ensuring that users can only access information appropriate to their authorization level.

For deployments handling protected health information (PHI), the platform may incorporate additional security measures to maintain compliance with regulations such as HIPAA or GDPR. This may include implementing audit logging mechanisms to track all data access and modifications, providing the capability to generate detailed compliance reports.

To support scalability and high availability, the healthcare knowledge platform may employ various architectural strategies. In some implementations, this may involve horizontal scaling through database sharding, distributing data across multiple servers based on partitioning keys. The system may utilize load balancing techniques to distribute query loads evenly across database instances, potentially improving response times and system resilience.

For disaster recovery and data durability, the platform may implement regular backup procedures and replication strategies. In some cases, this may involve maintaining geographically distributed replicas of critical data, allowing for rapid failover in the event of localized outages or data center failures.

The system may employ caching mechanisms at various levels to improve performance and reduce load on primary data stores. In some implementations, this may involve in-memory caching systems like Redis or Memcached to store frequently accessed data or intermediate computation results. The caching strategy may be dynamically adjusted based on usage patterns and system load to optimize resource utilization.

To manage the lifecycle of data within the platform, the system may implement data retention policies and archiving mechanisms. In some cases, this may involve moving less frequently accessed data to lower-cost storage tiers while maintaining rapid access to more recent or frequently used information. The platform may provide configurable data lifecycle management tools, allowing organizations to define retention periods and archiving rules based on their specific regulatory requirements and operational needs.

For handling real-time data streams, such as live clinical data feeds or user interaction events, the platform may incorporate stream processing technologies. In some implementations, this may involve using systems like Apache Kafka or Amazon Kinesis to ingest, buffer, and process high-volume data streams in real-time. The stream processing components may feed into both operational databases for immediate use and analytical data stores for long-term trend analysis and machine learning model training.

The healthcare knowledge platform may implement sophisticated query optimization techniques to ensure efficient data retrieval across its various data stores. In some cases, this may involve maintaining statistics on data distribution and query patterns, allowing the system to automatically adjust indexing strategies or query execution plans. The platform may employ query caching and result set materialization techniques to speed up repetitive or computationally expensive queries.

To support the platform's multi-agent architecture, the data storage and retrieval layer may provide specialized interfaces tailored to the needs of different agent types. In some implementations, this may involve creating abstraction layers that present unified views of data across multiple underlying storage systems, simplifying agent development and promoting modularity in the overall system design.

The platform may incorporate mechanisms for data versioning and provenance tracking, particularly for the dynamic healthcare knowledge graph and curated knowledge bases. In some cases, this may allow for temporal queries that reconstruct the state of medical knowledge at specific points in time, potentially supporting auditing, research, or educational use cases that require historical perspectives on evolving medical understanding.

For deployments in regulated environments, the system may implement data anonymization and de-identification pipelines as part of its data ingestion and storage processes. These pipelines may apply techniques such as k-anonymity or differential privacy to protect individual privacy while preserving the utility of data for analysis and knowledge extraction.

To facilitate interoperability with external healthcare systems, the data storage and retrieval mechanisms may support standard data exchange formats and protocols. In some implementations, this may involve providing FHIR-compliant APIs for clinical data access or supporting the ingestion and export of data in formats like HL7 or DICOM.

The healthcare knowledge platform may implement adaptive data management strategies that evolve based on usage patterns and system performance metrics. In some cases, this may involve using machine learning techniques to predict query patterns, preemptively optimize data layouts, or dynamically adjust caching strategies to maximize system efficiency.

Those skilled in the art will recognize that various modifications and variations can be made to the data storage and retrieval mechanisms without departing from the spirit and scope of the invention. The specific technologies, architectures, and optimization strategies may be adapted based on the scale of deployment, performance requirements, regulatory environment, and evolving best practices in healthcare data management and distributed systems design.

### System Integration and Interoperability

The healthcare knowledge platform may implement robust integration capabilities to facilitate seamless data exchange and interoperability with external clinical systems and health information networks. In some cases, the platform may utilize standardized healthcare interoperability protocols to ensure consistent and reliable communication with diverse healthcare IT ecosystems.

In some implementations, the system may support integration with Electronic Health Record (EHR) systems through the use of HL7 FHIR (Fast Healthcare Interoperability Resources) standards. FHIR may provide a modern, web-based approach to healthcare data exchange, potentially allowing the platform to retrieve and update patient information, clinical observations, and treatment plans in real-time.

The platform may also incorporate support for older healthcare messaging standards such as HL7 v2 or v3. In some cases, this backward compatibility may be crucial for integrating with legacy systems that have not yet adopted more recent interoperability standards. The system may employ message translation services to convert between different HL7 versions and FHIR, ensuring seamless communication across heterogeneous healthcare environments.

For integration with medical imaging systems, the platform may implement DICOM (Digital Imaging and Communications in Medicine) standard support. This may allow the system to query and retrieve medical images and associated metadata from Picture Archiving and Communication Systems (PACS) or Vendor Neutral Archives (VNA). In some implementations, the platform may incorporate DICOM viewer capabilities or integrate with third-party visualization tools to display retrieved images within its user interfaces.

To facilitate secure health information exchange across organizational boundaries, the platform may support integration with Health Information Exchange (HIE) networks. In some cases, this may involve implementing IHE (Integrating the Healthcare Enterprise) profiles such as Cross-Enterprise Document Sharing (XDS) for exchanging clinical documents or Patient Identifier Cross-Referencing (PIX) for managing patient identities across multiple systems.

The healthcare knowledge platform may implement support for standardized terminologies and code systems to ensure semantic interoperability. In some implementations, this may involve mapping internal concepts to widely used standards such as SNOMED CT, LOINC, or RxNorm. The system may maintain terminology services that can translate between different coding systems, potentially improving the accuracy of data integration and knowledge synthesis across diverse sources.

For integration with laboratory information systems, the platform may support LOINC (Logical Observation Identifiers Names and Codes) for standardized representation of laboratory tests and results. In some cases, the system may implement automated mapping between proprietary lab codes and LOINC, facilitating consistent interpretation of lab data across different healthcare organizations.

The platform may incorporate support for emerging interoperability standards such as SMART on FHIR or CDS Hooks. These standards may enable the development of pluggable applications or decision support services that can be seamlessly integrated into existing EHR workflows. In some implementations, the healthcare knowledge platform may expose its own capabilities as SMART on FHIR apps or CDS Hooks services, allowing its functionality to be embedded within other clinical systems.

To address privacy and security concerns in data exchange, the system may implement robust authentication and authorization mechanisms. In some cases, this may involve support for OAuth 2.0 and OpenID Connect protocols, allowing for secure, token-based authentication when integrating with external systems. The platform may also implement fine-grained access controls to ensure that only authorized users or systems can access specific data elements or functionalities.

For scenarios requiring bulk data transfer, the platform may support standards such as FHIR Bulk Data Access API. This capability may be particularly useful for population health management, analytics, or research use cases where large datasets need to be efficiently exchanged between systems.

The healthcare knowledge platform may implement flexible data mapping and transformation capabilities to handle variations in data formats and structures across different systems. In some cases, this may involve the use of Extract, Transform, Load (ETL) pipelines or more advanced data integration platforms that can perform complex data transformations and quality checks during the integration process.

To facilitate integration testing and validation, the platform may provide sandbox environments and test harnesses. These tools may allow developers and system integrators to simulate various integration scenarios and verify the correctness of data exchange processes before deploying to production environments.

The system may incorporate logging and auditing mechanisms specifically designed for tracking interoperability-related events. In some implementations, this may include detailed transaction logs for all data exchanges, potentially supporting troubleshooting, compliance reporting, and security monitoring in integrated healthcare environments.

For organizations operating in multiple geographic regions or jurisdictions, the platform may implement configurable interoperability profiles to address varying regulatory requirements and healthcare standards. This flexibility may allow the system to adapt to different national or regional healthcare IT frameworks while maintaining a consistent core architecture.

The healthcare knowledge platform may support both real-time and batch integration modes to accommodate different operational needs and system capabilities. Real-time integration may be crucial for scenarios requiring immediate data access or updates, while batch processing may be more suitable for large-scale data synchronization or analytical workloads.

In some cases, the platform may implement resilience and fault-tolerance mechanisms specifically designed for maintaining interoperability in the face of network issues or system outages. This may include features such as message queuing, retry logic, and eventual consistency models to ensure that data exchanges can recover gracefully from interruptions.

To streamline the integration process, the platform may provide developer-friendly APIs and software development kits (SDKs) for common programming languages. These tools may abstract away some of the complexities of healthcare interoperability standards, potentially reducing the learning curve for developers and accelerating integration projects.

The system may incorporate monitoring and analytics capabilities specifically focused on interoperability performance. In some implementations, this may include dashboards and alerts for tracking data exchange volumes, latencies, error rates, and other key performance indicators related to system integration and interoperability.

Those skilled in the art will recognize that various modifications and variations can be made to the integration and interoperability features without departing from the spirit and scope of the invention. The specific standards supported, integration architectures, and data exchange mechanisms may be adapted based on evolving healthcare IT landscapes, regulatory requirements, and technological advancements in health information exchange.

### Example Use Cases

The healthcare knowledge platform may support a variety of use cases across different healthcare scenarios. In some cases, the system may provide clinical decision support for practicing clinicians. For example, a cardiologist evaluating treatment options for a patient with complex coronary artery disease may utilize the platform to access the latest meta-analyses comparing drug-eluting stents to bare-metal stents in high-risk patients.

The conversational agent may clarify the query and gather additional context such as patient age and comorbidities. Specialized agents may then retrieve relevant studies from peer-reviewed literature and clinical guidelines. The dynamic knowledge graph may be queried to cross-reference relationships among treatment modalities, outcomes, and patient risk factors. A meta-analysis module may aggregate data from multiple studies, compute effect sizes, and generate visual summaries such as forest plots.

The system may provide the cardiologist with a synthesized summary of the evidence and tailored recommendations, potentially supporting more informed clinical decision-making. In some implementations, the platform may integrate with the hospital's electronic health record system to incorporate patient-specific data into its analysis and recommendations.

For patient-focused scenarios, the healthcare knowledge platform may facilitate physician referrals and appointment scheduling. A patient experiencing chronic knee pain may access the system seeking a referral to an medical specialist. The conversational agent may interact with the patient to confirm details such as location preferences and insurance coverage.

A physician referral module may query an integrated database to generate a list of qualified specialists matching the patient's criteria. The appointment scheduling component may offer available time slots and, upon patient selection, automatically book an appointment by interfacing with the chosen physician's scheduling system. This streamlined process may help ensure patients connect with appropriate care providers efficiently.

In educational contexts, the platform may support medical trainees preparing for examinations or expanding their clinical knowledge. A medical resident studying endocrinology may use the system to generate practice questions on diabetes management. The knowledge graph and large language models may be utilized to scan relevant concepts and craft multiple-choice and open-ended questions.

The question generation process may employ advanced natural language processing to ensure clinical accuracy and relevance. An adaptive learning system may track the resident's performance over time, adjusting the difficulty and focus of subsequent questions. The platform may provide customized question banks with detailed explanations and references to source literature, potentially enhancing the resident's study experience and clinical reasoning skills.

For research applications, the healthcare knowledge platform may assist in conducting systematic reviews and meta-analyses. A researcher investigating the efficacy of a new class of anticoagulants may utilize the system to streamline the review process. The platform may retrieve and index relevant clinical studies, guidelines, and articles based on specified inclusion criteria.

Specialized modules may apply statistical techniques to compute measures across selected studies and generate comprehensive summary reports. The dynamic knowledge graph may be leveraged to map relationships among study outcomes, patient demographics, and clinical endpoints. Visual outputs such as forest plots and funnel plots may be automatically generated to aid in data interpretation.

In some cases, the platform may support population health management initiatives. Healthcare administrators may use the system to analyze trends across large patient cohorts. The co-occurrence analysis capabilities may identify unexpected associations between diagnoses, treatments, and outcomes. This information may potentially inform the development of targeted interventions or care pathways.

For patient education purposes, the healthcare knowledge platform may generate personalized information materials. When a patient receives a new diagnosis, the system may create tailored educational content explaining the condition, typical treatment approaches, and self-management strategies. This content may be dynamically adjusted based on the patient's health literacy level, preferences, and specific clinical situation.

In emergency medicine scenarios, the platform may provide rapid access to critical information. An emergency department physician evaluating a patient with an unusual presentation may query the system for potential diagnoses. The multi-agent architecture may quickly retrieve and synthesize information from diverse sources, potentially including rare case reports or emerging research, to support timely and accurate clinical decision-making.

For medical device companies, the healthcare knowledge platform may assist in post-market surveillance activities. The system may continuously monitor and analyze real-world data sources for signals related to device performance or safety. Advanced natural language processing capabilities may extract relevant information from clinical notes, adverse event reports, and scientific literature, potentially enabling earlier detection of emerging issues.

In pharmaceutical research and development, the platform may support drug repurposing efforts. Researchers may utilize the system's knowledge graph and co-occurrence analysis features to identify potential new indications for existing medications. The platform may integrate data from diverse sources including genomics, proteomics, and clinical trials to suggest novel therapeutic applications.

For medical coding and billing purposes, the healthcare knowledge platform may provide assistance in selecting appropriate diagnostic and procedure codes. The system may analyze clinical documentation and suggest relevant ICD and CPT codes based on the described patient encounter. Co-occurrence data derived from large-scale EHR analysis may inform recommendations for commonly associated codes, potentially improving coding accuracy and completeness.

In telemedicine applications, the platform may enhance remote patient consultations. During a video visit, a healthcare provider may utilize the system's real-time information retrieval capabilities to access relevant clinical guidelines, drug interaction information, or patient education materials. The platform may also assist in documenting the encounter by generating structured note templates based on the conversation context.

For medical students engaged in problem-based learning curricula, the healthcare knowledge platform may serve as an interactive case study generator. The system may create complex, multi-stage clinical scenarios that evolve based on student decisions. As students work through these virtual cases, the platform may provide adaptive feedback, suggest relevant resources for further study, and assess clinical reasoning skills.

In public health surveillance, the platform may assist in early detection of disease outbreaks or emerging health trends. By continuously analyzing data from diverse sources including social media, news reports, and clinical data feeds, the system may identify anomalous patterns that warrant further investigation. Natural language processing capabilities may enable real-time processing of unstructured data to extract meaningful health-related signals.

For clinical trial recruitment, the healthcare knowledge platform may support matching potential participants with appropriate studies. The system may analyze patient records against trial eligibility criteria, considering factors such as diagnosis, biomarkers, medication history, and demographic information. This automated screening process may help researchers identify suitable candidates more efficiently, potentially accelerating the clinical trial process.

In some implementations, the platform may assist in the development and maintenance of clinical practice guidelines. The system may continuously monitor new research publications, meta-analyses, and real-world evidence relevant to specific clinical domains. This information may be synthesized and presented to guideline development committees, potentially supporting more timely updates to clinical recommendations based on emerging evidence.

Those skilled in the art will recognize that various modifications and variations can be made to these example use cases without departing from the spirit and scope of the invention. The specific applications and functionalities of the healthcare knowledge platform may be adapted based on evolving clinical needs, technological advancements, and changing healthcare delivery models.

### EXAMPLES

Aspects of the present teachings may be further understood in light of the following examples, which should not be construed as limiting the scope of the present teachings in any way.

### Example 1: Testing the Multi-Agent Orchestration for Complex Medical Queries

A clinical researcher tests the multi-agent orchestration capabilities of the healthcare knowledge platform by submitting a complex query: "What are the latest treatment recommendations for a 65-year-old patient with type 2 diabetes, hypertension, and early-stage chronic kidney disease?"

The researcher observes the following workflow:
1. The conversational agent processes the query and extracts key entities: age (65), conditions (type 2 diabetes, hypertension, chronic kidney disease), and query type (treatment recommendations).
2. The directing agent routes sub-queries to specialized agents:
   a. Knowledge Graph Query Agent: Retrieves relevant disease relationships and potential drug interactions.
   b. Literature Retrieval Agent: Searches for recent clinical guidelines and meta-analyses.
   c. EHR Data Agent: Analyzes anonymized patient data for common treatment patterns.
   d. Co-occurrence Agent: Identifies frequently associated complications or comorbidities.
3. The summarization agent consolidates the retrieved information, generating a structured report with treatment options, potential risks, and supporting evidence.
4. The LLM agent, using retrieval-augmented generation, produces a natural language summary tailored for clinical use.

### Expected Results:

The system generates a comprehensive, evidence-based treatment plan within 30 seconds. The plan includes:
- First-line and alternative medication options, with dosage adjustments for kidney function
- Lifestyle modification recommendations
- Monitoring parameters for blood glucose, blood pressure, and kidney function
- Potential drug interactions and contraindications
- References to specific clinical guidelines and recent studies

The researcher evaluates the output for accuracy, relevance, and completeness, comparing it to manual literature reviews and expert consensus. The multi-agent approach is expected to provide more comprehensive and up-to-date recommendations than single-source or rule-based systems.

### Example 2: Evaluating the Dynamic Healthcare Knowledge Graph

A medical informaticist tests the platform's ability to maintain an up-to-date and contextually relevant knowledge graph. The test focuses on a rapidly evolving area of medicine: SARS-CoV-2 variants and vaccine efficacy.

### Test Protocol:

1. The informaticist seeds the knowledge graph with initial data on SARS-CoV-2 and known variants as of January 2023.
2. Over a simulated 6-month period, the system ingests new information from:
   a. PubMed and preprint servers (e.g., medRxiv) for new research articles
   b. WHO and CDC websites for updated guidelines
   c. GISAID database for new variant sequences
   d. Anonymized EHR data for real-world vaccine effectiveness
3. At monthly intervals, the informaticist queries the system for:
   a. Current dominant variants
   b. Vaccine efficacy against these variants
   c. Updated booster recommendations
4. The informaticist tracks:
   a. Speed of knowledge graph updates following new publications
   b. Accuracy of information compared to manual expert curation
   c. The system's ability to resolve conflicting information from different sources

### Expected Results:

- The knowledge graph continuously expands, with new nodes and edges added within 24 hours of publication for high-impact findings.
- Queries at each time point reflect the most current understanding of variant prevalence and vaccine efficacy.
- The system successfully integrates information across multiple sources, providing a more comprehensive view than any single database.
- When conflicting data emerges, the system presents multiple viewpoints with associated confidence levels based on study quality and consensus.
- By the end of the 6-month period, the knowledge graph contains a rich, interconnected representation of SARS-CoV-2 variants, their characteristics, and their implications for vaccination strategies.

### Example 3: Assessing Adaptive Question Generation for Medical Education

A medical education specialist evaluates the platform's ability to generate and adapt question banks for training medical residents in cardiology.

### Test Protocol:

1. The specialist defines initial parameters:
   - Topic: Acute Coronary Syndrome (ACS)
   - Target learners: First-year cardiology residents
   - Initial difficulty: Moderate
      - Question types: Multiple choice, case vignettes, and image interpretation
2. The system generates an initial set of 50 questions based on the knowledge graph and current guidelines for ACS management.
3. Twenty residents take the initial question set as a pre-test.
4. Over eight weeks, residents use the platform for weekly assessments. The specialist monitors:
   a. Question difficulty adaptation based on individual and group performance
   b. Integration of new content as it appears in the knowledge graph
   c. Generation of questions targeting identified knowledge gaps
5. At the end of eight weeks, residents take a post-test generated by the system.

### Expected Results:

- Initial question set shows high relevance to current ACS guidelines and clinical practice.
- Question difficulty adapts over time:
   High-performing residents receive progressively more challenging questions
   Struggling residents receive more foundational questions with detailed explanations
      - New content (e.g., a newly published ACS risk stratification tool) is incorporated into questions within one week of addition to the knowledge graph.
      - The system identifies common misconceptions across the resident group and generates targeted questions to address these gaps.
      - Post-test results show significant improvement in resident knowledge and clinical reasoning skills related to ACS.
      - Residents report high satisfaction with the adaptive nature of the questions and the immediate, detailed feedback provided.

The medical education specialist compares the adaptive question bank's effectiveness to traditional static question banks, evaluating factors such as knowledge retention, critical thinking development, and alignment with current best practices in ACS management.

### Other Embodiments

The detailed description set-forth above is provided to aid those skilled in the art in practicing the present invention. However, the invention described and claimed herein is not to be limited in scope by the specific embodiments herein disclosed because these embodiments are intended as illustration of several aspects of the invention. Any equivalent embodiments are intended to be within the scope of this invention. Indeed, various modifications of the invention in addition to those shown and described herein will become apparent to those skilled in the art from the foregoing description which do not depart from the spirit or scope of the present inventive discovery. Such modifications are also intended to fall within the scope of the appended claims.

### References Cited

All publications, patents, patent applications and other references cited in this application are incorporated herein by reference in their entirety for all purposes to the same extent as if each individual publication, patent, patent application or other reference was specifically and individually indicated to be incorporated by reference in its entirety for all purposes. Citation of a reference herein shall not be construed as an admission that such is prior art to the present invention.

## Claims

1. A healthcare knowledge platform, comprising:
a user interface configured to receive queries;
a multi-agent processing module having a directing agent, a conversational agent, and specialized agents for knowledge retrieval, summarization, and co-occurrence-based recommendations;
a knowledge retrieval engine configured to access at least one source selected from the group consisting of large language models, curated healthcare databases, peer-reviewed journals, textbooks, and electronic health record (EHR)-based co-occurrence data;
a summarizing engine configured to generate summarized content or perform live meta-analyses of retrieved documents;
a dynamically expandable healthcare knowledge graph storing medical concepts, relationships, metadata, and co-occurrence frequencies; and
a database for storing user queries, retrieved data, summaries, co-occurrence metrics, and system analytics.

2. The healthcare knowledge platform of claim 1, wherein the multi-agent processing module operates in parallel, providing an initial low-latency response while a more comprehensive analysis is performed by specialized agents that incorporate dual co-occurrence recommendations.

3. The healthcare knowledge platform of claim 2, further comprising an educational module configured to automatically generate healthcare training questions tailored to user-selected topics or difficulty levels, wherein each question is linked to at least one node in the healthcare knowledge graph and optionally references co-occurrence-based expansions derived from EHR data.

4. The healthcare knowledge platform of claim 3, wherein the healthcare knowledge graph is updated dynamically based on newly published research, clinical guidelines, user feedback, and combined co-occurrence data, ensuring the platform remains current with both recognized evidence and real-world usage patterns.

5. A method for providing health-related information to users via a healthcare knowledge platform, comprising:
receiving, by a conversational agent, a user query through a user interface;
analyzing said query via a directing agent configured to determine relevant specialized agent(s) to invoke based on query type and user context and initiate sub-queries to one or more agents selected from the group consisting of a lab data agent, a visual data agent, a summarizing agent, a co-occurrence-based recommendation agent, a meta-analysis agent, and a question generation agent;
retrieving data from at least one source selected from the group consisting of a large language model, a curated healthcare knowledge base, peer-reviewed journals, textbooks, and statistical co-occurrence-based data derived from electronic health records (EHRs);
querying a healthcare knowledge graph to determine semantic relationships among medical concepts relevant to the user query; and
returning a consolidated response to the user via the conversational agent, wherein recommended expansions or additional search terms are generated through combined statistical and semantic co-occurrence analytics.

6. The method of claim 5, wherein analyzing said query further comprises computing semantic similarity between the retrieved data and the user query based on:
co-occurrence frequency metrics derived from aggregated, anonymized EHR data, and
semantic relationships stored in the healthcare knowledge graph,
to provide context-aware results beyond purely frequency-based or purely ontology-based methods.

7. The method of claim 5, further comprising generating and maintaining the healthcare knowledge graph of medical concepts and typed relationships, wherein the graph is updated in near real time based on:
newly published research and clinical guidelines,
user or domain-expert feedback, and
co-occurrence-based recommendations that merge statistical EHR data with semantic graph structures,
thereby reflecting evolving clinical practices and evidence.

8. The method of claim 7, wherein the platform automatically generates educational question banks for healthcare trainees by:
analyzing the healthcare knowledge graph to identify key concepts or relationships,
applying co-occurrence data to rank topics by relevance or underexplored frequency, and
using a large language model for automated question generation to produce multiple-choice, short-answer, or case-based questions at varying difficulty levels.

9. A non-transitory computer-readable medium storing instructions which, when executed by one or more processors, cause the one or more processors to perform a method for facilitating healthcare decision support, comprising:
receiving a real-time user query;
dynamically accessing data from live clinical sources, historical EHR data, and curated medical databases;
processing the query using a multi-agent system orchestrated with a dynamically updated healthcare knowledge graph;
generating personalized recommendations or educational content based on combined statistical and semantic co-occurrence; and
providing the results to the user through an interactive, secure interface, wherein the output includes adaptive question banks and real-time meta-analyses.

10. The non-transitory computer-readable medium of claim 9, wherein the method further comprises:
automatically updating the healthcare knowledge graph by incorporating new research articles, clinical guidelines, and real-time clinical data, wherein machine learning algorithms continuously analyze the incoming data to adjust semantic relationships among medical entities and update co-occurrence scores.

11. The non-transitory computer-readable medium of claim 10, wherein the method further comprises:
detecting anomalies in co-occurrence patterns by comparing the statistical frequency of co-occurring medical codes or search terms against historical baselines, and automatically generating alerts for healthcare providers when such anomalies are detected, thereby supporting early identification of emerging trends or errors.

12. The non-transitory computer-readable medium of claim 9, wherein the method further comprises:
providing physician referral recommendations to a patient based on one or more input parameters selected from the group consisting of required medical specialty, geographic location, insurance coverage, and patient preferences, wherein the system cross-references a physician database to identify matching healthcare providers and merges that information with knowledge graph data on provider expertise.

13. A method of generating educational content for healthcare trainees, comprising:
analyzing a healthcare knowledge graph to identify key concepts or relationships;
applying co-occurrence data to rank topics by relevance or underexplored frequency, wherein the co-occurrence data is derived from both statistical analysis of electronic health records and semantic relationships within the healthcare knowledge graph; and
using a large language model for automated question generation to produce multiple-choice, short-answer, or case-based questions at varying difficulty levels, wherein each generated question is linked to at least one node in the healthcare knowledge graph.

14. The method of claim 13, wherein the difficulty levels of the generated questions are dynamically adjusted based on trainee performance data, and wherein the method further comprises:
tracking individual trainee responses to generated questions;
analyzing patterns of correct and incorrect answers; and
automatically adjusting the difficulty and focus of subsequent questions to target identified knowledge gaps.

15. The method of claim 14, wherein the large language model is retrieval-augmented with context from the healthcare knowledge graph and recent medical literature, and wherein the method further comprises:
generating detailed explanations for each question, including references to relevant nodes in the knowledge graph and citations to supporting literature; and
providing adaptive feedback to trainees based on their responses, incorporating co-occurrence data to suggest related topics for further study.
